# EUROPEAN PATENT APPLICATION

(11) **EP 0 722 727 A1**
(43) Date of publication of application: **24.07.1996**
(21) Application number: 96300345.4
(22) Date of filing: 17.01.1996
(51) Int. Cl.: A61K 31/34, A61K 31/40, A61K 31/445, A61K 31/535

(54) **Benzofuran derivatives for inhibiting uterine fibroid disease**

(30) Priority: 20.01.1995 US 376956
(71) Applicant: ELI LILLY AND COMPANY, Indianapolis, Indiana 46285 (US)
(72) Inventor: Fontana, Stephen A., Martinsville, Indiana 46151 (US)
(74) Representative: Tapping, Kenneth George

(57) **Abstract**

The present invention provides a method for inhibiting uterine fibroid disease comprising administering to a woman in need of treatment an effective amount of a compound of formula I 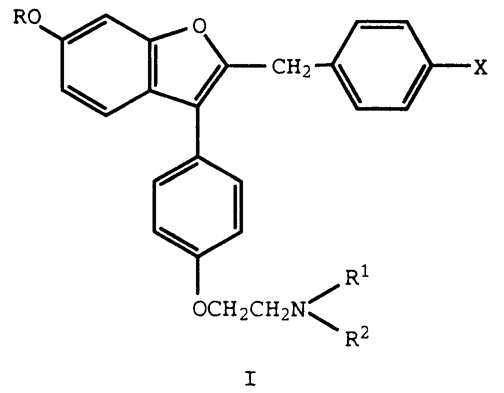
wherein
R is hydrogen or methyl;
R¹ and R² each are methyl or ethy, or R¹ and R² together with the nitrogen atom to which they are attached represent a saturated heterocyclic group; and
X is bromo, chloro, fluoro, or hydrogen; or a pharmaceutically acceptable salt thereof.

## Description

The present invention relates to the discovery that a group of benzofuran derivatives are useful for inhibiting uterine fibroid disease in women.

Uterine fibroid disease (uterine fibrosis) is an old and ever present clinical problem which goes under a variety of names, including uterine hypertrophy, uterine lieomyomata, myometrial hypertrophy, fibrosis uteri, and fibrotic metritis. Essentially, uterine fibroid disease is a condition where there is an inappropriate deposition of fibroid tissue on the wall of the uterus.

This condition is a cause of dysmenorrhea and infertility in women. The exact cause of this condition is poorly understood but evidence suggests that it is an inappropriate response of fibroid tissue to estrogen. Such a condition has been produced in rabbits by daily administrations of estrogen for 3 months. In guinea pigs, the condition has been produced by daily administration of estrogen for four months. Further, in rats, estrogen causes similar hypertrophy.

The most common treatment of uterine fibroid disease involves surgical procedures which are both costly and sometimes a source of complications such as the formation of abdominal adhesions and infections. In some patients, initial surgery is only a temporary treatment and the fibroids regrow. In those cases, a hysterectomy is performed which effectively ends the fibroids, but also the reproductive life of the patient. Also, gonadotropin releasing hormone antagonists may be administered, but their use is tempered by the fact they can lead to osteoporosis.

Thus, new methods for treating uterine fibroid disease in women are desirable.

The present invention relates to methods for inhibiting uterine fibroid disease comprising administering to a woman in need of treatment an effective amount of a compound of formula I 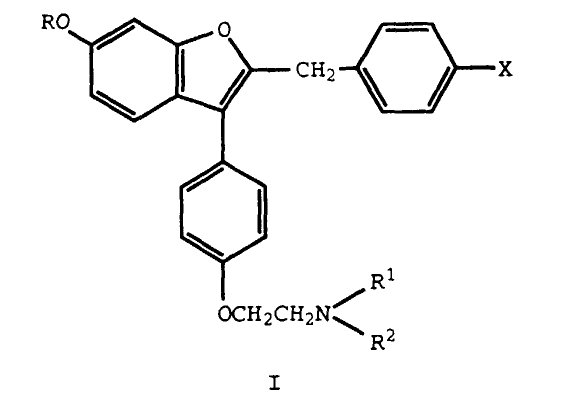 wherein
R is hydrogen or methyl;
R¹ and R² each are methyl or ethy, or R¹ and R² together with the nitrogen atom to which they are attached represent a saturated heterocyclic group; and
X is bromo, chloro, fluoro, or hydrogen; or a pharmaceutically acceptable salt thereof.

The present invention concerns methods for inhibiting uterine fibroid disease in women. The term "inhibit" is defined to include its generally accepted meaning which includes prophylactically treating a subject from incurring one or more of these disease states, holding in check the symptoms of such a disease state, and/or treating such symptoms. Thus, the present methods include both medical therapeutic and/or prophylactic treatment, as appropriate.

The methods of this invention are practiced by administering to a woman in need of treatment an effective amount of a compound of formula I 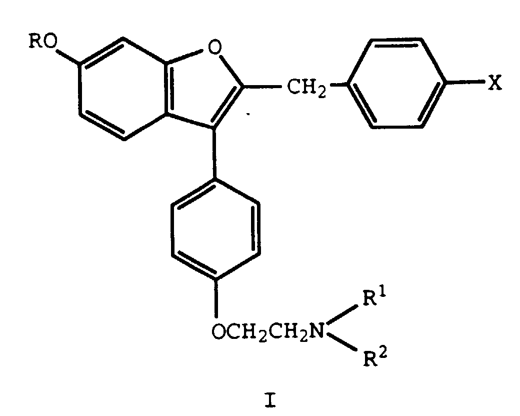 wherein
R is hydrogen or methyl;
R¹ and R² each are methyl or ethy, or R¹ and R² together with the nitrogen atom to which they are attached represent a saturated heterocyclic group; and
X is bromo, chloro, fluoro, or hydrogen;
or a pharmaceutically acceptable salt thereof.

Compounds of formula I are known in the art and essentially are prepared according to established or analogous procedures, such as those detailed in U.S. Pat. No. 5,354,861, which is herein incorporated by reference.

Preferred formula I compounds are those in which R¹ and R² independently are methyl or ethyl or, when taken together with the nitrogen atom to which they are attached represent a pyrrolidino, piperidino, or morpholino group.

Representative preferred compounds are as follows:
2-(p-chlorobenzyl)-3-[p-(2-dimethylaminoethoxy) phenyl]-6-methoxy-benzo[b]furan;
2-(p-chlorobenzyl)-3-[p-(2-pyrrolidinoethoxy) phenyl]-6-methoxy-benzo[b]furan;
2-(p-chlorobenzyl)-3-[p-(2-piperidinoethoxy)phenyl]-6-methoxy-benzo[b]furan;
2-(p-chlorobenzyl)-3-[p-(2-morpholinoethoxy)phenyl]-6-methoxy-benzo[b]furan;
2-(p-fluorobenzyl)-3-[p-(2-dimethylaminoethoxy) phenyl]-6-methoxy-benzo[b]furan;
2-(p-fluorobenzyl)-3-[p-(2-pyrrolidinoethoxy)phenyl]-6-methoxy-benzo[b]furan;
2-(p-fluorobenzyl)-3-[p-(2-piperidinoethoxy)phenyl]-6-methoxy-benzo[b]furan; and
2-(p-fluorobenzyl)-3-[p-(2-morpholinoethoxy)phenyl]-6-methoxy-benzo[b]furan.

Although the free-base form of formula I compounds can be used in the methods of the present invention, it is preferred to prepare and use a pharmaceutically acceptable salt form. Thus, the compounds used in the methods of this invention form pharmaceutically acceptable acid salts with a wide variety of inorganic and, preferrably, organic acids, and include the physiologically acceptable salts which are often used in pharmaceutical chemistry. Such salts are also part of this invention. Typical inorganic acids used to form such salts include hydrochloric, hydrobromic, hydroiodic, nitric, sulfuric, phosphoric, hypophosphoric, and the like. Salts derived from organic acids, such as aliphatic mono and dicarboxylic acids, phenyl substituted alkanoic acids, hydroxyalkanoic and hydroxyalkandioic acids, aromatic acids, aliphatic and aromatic sulfonic acids, may also be used. Such pharmaceutically acceptable salts thus include acetate, phenylacetate, trifluoroacetate, acrylate, ascorbate, benzoate, chlorobenzoate, dinitrobenzoate, hydroxybenzoate, methoxybenzoate, methylbenzoate, o-acetoxybenzoate, naphthalene-2-benzoate, bromide, isobutyrate, phenylbutyrate, β-hydroxybutyrate, butyne-1,4-dioate, hexyne-1,4-dioate, caprate, caprylate, chloride, cinnamate, citrate, formate, fumarate, glycollate, heptanoate, hippurate, lactate, malate, maleate, hydroxymaleate, malonate, mandelate, mesylate, nicotinate, isonicotinate, nitrate, oxalate, phthalate, terephthalate, phosphate, monohydrogenphosphate, dihydrogenphosphate, metaphosphate, pyrophosphate, propiolate, propionate, phenylpropionate, salicylate, sebacate, succinate, suberate, sulfate, bisulfate, pyrosulfate, sulfite, bisulfite, sulfonate, benzenesulfonate, p-bromophenylsulfonate, chlorobenzene-sulfonate, ethanesulfonate, 2-hydroxyethanesulfonate, methanesulfonate, naphthalene-1-sulfonate, naphthalene-2-sulfonate, p-toluenesulfonate, xylenesulfonate, tartarate, and the like.

The pharmaceutically acceptable acid addition salts are typically formed by reacting a compound of formula I with an equimolar or excess amount of acid. The reactants are generally combined in a mutual solvent such as diethyl ether or benzene. The salt normally precipitates out of solution within about one hour to 10 days and can be isolated by filtration or the solvent can be stripped off by conventional means.

The pharmaceutically acceptable salts of formula I compounds generally have enhanced solubility characteristics compared to the compound from which they are derived, and thus are often more amenable to formulation as liquids or emulsions.

Once prepared, the free base or salt form of formula I compounds can be administered to an individual in need of treatment for the methods herein described. The following nonlimiting test examples illustrate the methods of the present invention.

In the examples illustrating the methods, a postmenopausal model is used to determine the effect of different treatments upon test animal uteri.

Seventy-five day old female Sprague Dawley rats (weight range of 200 to 250g) are obtained from Charles River Laboratories (Portage, MI). The animals are either bilaterally ovariectomized (OVX) or exposed to a Sham surgical procedure at Charles River Laboratories, and then shipped after one week. Upon arrival, they are housed in metal hanging cages in groups of 3 or 4 per cage and have ad *libitum* access to food (calcium content approximately 0.5%) and water for one week. Room temperature is maintained at 22.2° ± 1.7° C with a minimum relative humidity of 40%. The photoperiod in the room is 12 hours light and 12 hours dark.

Dosing Regimen Tissue Collection. After a one week acclimation period (therefore, two weeks post-OVX) daily dosing with test compound is initiated. 17α-ethynyl estradiol and the test compound are given orally, unless otherwise stated, as a suspension in 20% cyclodextrin. Animals are dosed daily for 4 days. Following the dosing regimen, animals are weighed and anesthetized with a ketamine: Xylazine (2:1, V:V) mixture and a blood sample is collected by cardiac puncture. The animals are then sacrificed by asphyxiation with CO₂, the uterus is removed through a midline incision, and a wet uterine weight is determined.

### Uterine Fibrosis Test Procedures

### Test 1

Between 3 and 20 women having uterine fibrosis are administered a compound of the present invention. The amount of compound administered is from 0.1 to 1000 mg/day, and the period of administration is 3 months.

The women are observed during the period of administration, and up to 3 months after discontinuance of administration, for effects on uterine fibrosis.

### Test 2

The same procedure is used as in Test 1, except the period of administration is 6 months.

### Test 3

The same procedure is used as in Test 1, except the period of administration is 1 year.

### Test 4

### A. Induction of fibroid tumors in guinea pig.

Prolonged estrogen stimulation is used to induce leiomyomata in sexually mature female guinea pigs. Animals are dosed with estradiol 3-5 times per week by injection for 2-4 months or until tumors arise. Treatments consisting of a compound of the invention or vehicle is administered daily for 3-16 weeks and then animals are sacrificed and the uteri harvested and analyzed for tumor regression.

### B. Implantation of human uterine fibroid tissue in nude mice.

Tissue from human leiomyomas are implanted into the peritoneal cavity and or uterine myometrium of sexually mature, castrated, female, nude mice. Exogenous estrogen are supplied to induce growth of the explanted tissue. In some cases, the harvested tumor cells are cultured *in vitro* prior to implantation. Treatment consisting of a compound of the present invention or vehicle is supplied by gastric lavage on a daily basis for 3-16 weeks and implants are removed and measured for growth or regression. At the time of sacrifice, the uteri is harvested to assess the status of the organ.

### Test 5

A. Tissue from human uterine fibroid tumors is harvested and maintained, *in vitro,* as primary nontransformed cultures. Surgical specimens are pushed through a sterile mesh or sieve, or alternately teased apart from surrounding tissue to produce a single cell suspension. Cells are maintained in media containing 10% serum and antibiotic. Rates of growth in the presence and absence of estrogen are determined. Cells are assayed for their ability to produce complement component C3 and their response to growth factors and growth hormone. *In vitro* cultures are assessed for their proliferative response following treatment with progestins, GnRH, a compound of the present invention and vehicle. Levels of steroid hormone receptors are assessed weekly to determine whether important cell characteristics are maintained *in vitro.* Tissue from 5-25 patients are utilized.

Activity in at least one of the above tests indicates the compounds of the present invention are of potential in the treatment of uterine fibrosis.

For the methods of the present invention, compounds of Formula I are administered continuously, from 1 to 4 times daily. However, cyclical therapy may especially be useful in the treatment of endometriosis or may be used acutely during painful attacks of the disease.

As used herein, the term "effective amount" means an amount of compound of the methods of the present invention which is capable of inhibiting the symptoms of the pathological conditions herein described. The specific dose of a compound administered according to this invention will, of course, be determined by the particular circumstances surrounding the case including, for example, the compound administered, the route of administration, the state of being of the patient, and the severity of the pathological condition being treated. A typical daily dose will contain a nontoxic dosage level of from about 0.25 mg to about 400 mg/day of a compound of the present invention. Preferred daily doses generally will be from about 1 mg to about 20 mg/day.

The compounds of this invention can be administered by a variety of routes including oral, rectal, transdermal, subucutaneus, intravenous, intramuscular, and intranasal. These compounds preferably are formulated prior to administration, the selection of which will be decided by the attending physician. Typically, a formula I compound, or a pharmaceutically acceptable salt thereof, is combined with a pharmaceutically acceptable carrier, diluent or excipient to form a pharmaceutical formulation.

The total active ingredients in such formulations comprises from 0.1% to 99.9% by weight of the formulation. By "pharmaceutically acceptable" it is meant the carrier, diluent, excipients, and/or salt must be compatible with the other ingredients of the formulation, and not deleterious to the recipient thereof.

Pharmaceutical formulations containing a compound of formula I can be prepared by procedures known in the art using well known and readily available ingredients. For example, the compounds of formula I can be formulated with common excipients, diluents, or carriers, and formed into tablets, capsules, suspensions, powders, and the like. Examples of excipients, diluents, and carriers that are suitable for such formulations include the following: fillers and extenders such as starch, sugars, mannitol, and silicic derivatives; binding agents such as carboxymethyl cellulose and other cellulose derivatives, alginates, gelatin, and polyvinyl-pyrrolidone; moisturizing agents such as glycerol; disintegrating agents such as calcium carbonate and sodium bicarbonate; agents for retarding dissolution such as paraffin; resorption accelerators such as quaternary ammonium compounds; surface active agents such as cetyl alcohol, glycerol monostearate; adsorptive carriers such as kaolin and bentonite; and lubricants such as talc, calcium and magnesium stearate, and solid polyethyl glycols.

The compounds also can be formulated as elixirs or solutions for convenient oral administration or as solutions appropriate for parenteral administration, for example, by intramuscular, subcutaneous or intravenous routes.

Additionally, the compounds are well suited to formulation as sustained release dosage forms and the like. The formulations can be so constituted that they release the active ingredient only or preferably in a particular physiological location, possibly over a period of time. The coatings, envelopes, and protective matrices may be made, for example, from polymeric substances or waxes.

Compounds of formula I generally will be administered in a convenient formulation. The following formulation examples only are illustrative and are not intended to limit the scope of the present invention.

### Formulations

In the formulations which follow, "active ingredient" means a compound of formula I, or a salt thereof.

### Formulation 1: Gelatin Capsules

Hard gelatin capsules are prepared using the following:

| Ingredient | Quantity (mg/capsule) |
|---|---|
| Active ingredient | 0.25 - 400 |
| Starch, NF | 0 - 650 |
| Starch flowable powder | 0 - 50 |
| Silicone fluid 350 centistokes | 0 - 15 |

The formulation above may be changed in compliance with the reasonable variations provided.

A tablet formulation is prepared using the ingredients below:

### Formulation 2: Tablets

| Ingredient | Quantity (mg/tablet) |
|---|---|
| Active ingredient | 0.25 - 400 |
| Cellulose, microcrystalline | 200 - 650 |
| Silicon dioxide, fumed | 10 - 650 |
| Stearate acid | 5 - 15 |

The components are blended and compressed to form tablets.

Alternatively, tablets each containing 0.25 - 400 mg of active ingredient are made up as follows:

### Formulation 3: Tablets

| Ingredient | Quantity (mg/tablet) |
|---|---|
| Active ingredient | 0.25 - 400 |
| Starch | 45 |
| Cellulose, microcrystalline | 35 |
| Polyvinylpyrrolidone (as 10% solution in water) | 4 |
| Sodium carboxymethyl cellulose | 4.5 |
| Magnesium stearate | 0.5 |
| Talc | 1 |

The active ingredient, starch, and cellulose are passed through a No. 45 mesh U.S. sieve and mixed thoroughly. The solution of polyvinylpyrrolidone is mixed with the resultant powders which are then passed through a No. 14 mesh U.S. sieve. The granules so produced are dried at 50°-60° C and passed through a No. 18 mesh U.S. sieve. The sodium carboxymethyl starch, magnesium stearate, and talc, previously passed through a No. 60 U.S. sieve, are then added to the granules which, after mixing, are compressed on a tablet machine to yield tablets.

Suspensions each containing 0.25 - 400 mg of medicament per 5 ml dose are made as follows:

### Formulation 4: Suspensions

| Ingredient | Quantity (mg/5 ml) |
|---|---|
| Active ingredient | 0.25 - 400 mg |
| Sodium carboxymethyl cellulose | 50 mg |
| Syrup | 1.25 mg |
| Benzoic acid solution | 0.10 mL |
| Flavor | q.v. |
| Color | q.v. |
| Purified water to | 5 mL |

The medicament is passed through a No. 45 mesh U.S. sieve and mixed with the sodium carboxymethyl cellulose and syrup to form a smooth paste. The benzoic acid solution, flavor, and color are diluted with some of the water and added, with stirring. Sufficient water is then added to produce the required volume.

An aerosol solution is prepared containing the following ingredients:

### Formulation 5: Aerosol

| Ingredient | Quantity (% by weight) |
|---|---|
| Active ingredient | 0.25 |
| Ethanol | 25.75 |
| Propellant 22 (Chlorodifluoromethane) | 70.00 |

The active ingredient is mixed with ethanol and the mixture added to a portion of the propellant 22, cooled to 30° C, and transferred to a filling device. The required amount is then fed to a stainless steel container and diluted with the remaining propellant. The valve units are then fitted to the container.

Suppositories are prepared as follows:

### Formulation 6: Suppositories

| Ingredient | Quantity (mg/suppository) |
|---|---|
| Active ingredient | 250 |
| Saturated fatty acid glycerides | 2,000 |

The active ingredient is passed through a No. 60 mesh U.S. sieve and suspended in the saturated fatty acid glycerides previously melted using the minimal necessary heat. The mixture is then poured into a suppository mold of nominal 2 g capacity and allowed to cool.
An intravenous formulation is prepared as follows:

### Formulation 7: Intravenous Solution

| Ingredient | Quantity |
|---|---|
| Active ingredient | 20 mg |
| Isotonic saline | 1,000 mL |

The solution of the above ingredients is intravenously administered to a patient at a rate of about 1 mL per minute.

## Claims

1. The use of a compound of formula I 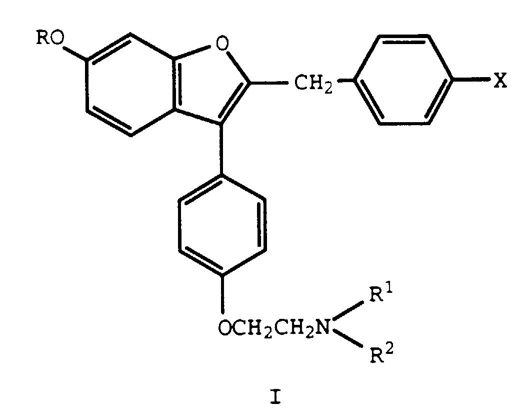 wherein
R is hydrogen or methyl;
R¹ and R² each are methyl or ethyl, or R¹ and R² together with the nitrogen atom to which they are attached represent a saturated heterocyclic group; and
X is bromo, chloro, fluoro, or hydrogen;
or a pharmaceutically acceptable salt thereof in the preparation of a medicament useful for inhibiting uterine fibroid disease.

2. The use according to Claim 1 wherein R¹ and R² of said formula I compound independently are methyl or ethyl, or together with the nitrogen atom to which they are attached represent a pyrrolidino, piperidino, or morpholino group.

3. The use according to Claim 2 wherein said formula I compound is 2-(p-chlorobenzyl)-3-[p-(2-dimethylaminoethoxy)phenyl]-6-methoxy-benzo[b]furan.

4. The use according to Claim 2 wherein said formula I compound is 2-(p-chlorobenzyl)-3-[p-(2-pyrrolidinoethoxy)phenyl]-6-methoxy-benzo[b]furan.

5. The use according to Claim 2 wherein said formula I compound is 2-(p-chlorobenzyl)-3-[p-(2-piperidinoethoxy)phenyl]-6-methoxy-benzo[b]furan.

6. The use according to Claim 2 wherein said formula I compound is 2-(p-fluorobenzyl)-3-[p-(2-dimethylaminoethoxy)phenyl]-6-methoxy-benzo[b]furan.

7. The use according to Claim 2 wherein said formula I compound is 2-(p-fluorobenzyl)-3-[p-(2-pyrrolidinoethoxy)phenyl]-6-methoxy-benzo [b] furan.

8. The use according to Claim 2 wherein said formula I compound is 2-(p-fluorobenzyl)-3-[p-(2-piperidinoethoxy)phenyl]-6-methoxy-benzo[b]furan.

9. The use according to any one of claims 1 to 8, and further comprising administering to said mammal an effective amount of estrogen.
